## Europäisches Patentamt
## European Patent Office
## Office européen des brevets

(11) Veröffentlichungsnummer: **0 291 642**
**B1**

(12) **EUROPÄISCHE PATENTSCHRIFT**

(45) Veröffentlichungstag der Patentschrift:
13.06.90

(51) Int. Cl.⁵: **C07C 37/84**, C07C 39/07

(21) Anmeldenummer: 88102888.0

(22) Anmeldetag: 26.02.88

(54) Verfahren zur Gewinnung von 3,4-Dimethylphenol.

(30) Priorität: 22.05.87 DE 3717282

(43) Veröffentlichungstag der Anmeldung:
23.11.88 Patentblatt 88/47

(45) Bekanntmachung des Hinweises auf die Patenterteilung:
13.06.90 Patentblatt 90/24

(84) Benannte Vertragsstaaten:
DE FR GB IT NL

(56) Entgegenhaltungen:
EP-A- 0 112 615
DE-A- 2 927 810
DE-B- 1 234 226
US-A- 4 491 677

CHEMICAL ABSTRACTS, Bd. 90, Nr. 11, 12. März 1979, Seite 573, Abstract Nr. 86912s; Columbus, Ohio, US; Y. MURATA et al.: "Study on an extraneous agent for adductive crystallization. The case of forming an intermolecular compound."
METHODEN DER ORGANISCHEN CHEMIE (HOUBEN-WEYL),Band VI/Ic, Teil 2, 4. Auflage, Georg Thieme Verlag, 1976, Stuttgart, DE;

(73) Patentinhaber: RÜTGERSWERKE AKTIENGESELLSCHAFT, Mainzer Landstrasse 217, D-6000 Frankfurt am Main 1(DE)

(72) Erfinder: Hansmann, Udo, Waltroperstrasse 75, D-4714 Selm-Bork(DE)
Erfinder: Spengler, Hans, Dr., Windmühlenberg 3, D-4716 Olfen(DE)
Erfinder: Marrett, Rolf, Eichenweg 17, D-4620 Castrop-Rauxel(DE)

**Beschreibung**

Die Erfindung betrifft ein Verfahren zur Gewinnung von 3,4-Dimethylphenol aus der bei der destillativen Aufarbeitung des Rohphenols anfallenden, p-Isopropylphenol enthalten Dimethylphenol-Fraktion.

Aufgrund seiner starken bakterientötenden Wirkung wird 3,4-Dimethylphenol bei der Herstellung von Desinfektions- und Schädlingsbekämpfungsmitteln verwendet. Auch in der Polymerchemie findet es, gegebenfalls nach Oxidation Verwendung. Als Rohstoffquelle für 3,4-Dimethylphenol dient der Steinkohlenteer, der selbst nur wenig p-Isopropylphenol enthält. Durch die gemeinsame Aufarbeitung von Rohphenol aus Steinkohlenteer und anderen Phenole enthaltenden Rückständen gelangen erhebliche Mengen an p-Isopropylphenol in die Dimethylphenol-Fraktion. Wegen der geringen Siedepunktsdifferenz von etwa 2°C lassen sich die beiden Stoffe destillativ bei wirtschaftlich vertretbarem Aufwand nicht trennen. Die Differenz der Schmelzpunkte beider Stoffe beträgt etwa 4°C. Aus Versuchen ist bekannt, daß die Gewinnung von 3,4-Dimethylphenol durch Kristallisation nur möglich ist, wenn das Konzentrationsverhältnis 3,4-Dimethylphenol zu p-Isoprophylphenol größer als 1,5, oder besser größer als 2 ist, und die Menge an Begleitkomponenten 10 % nicht überschreitet. Diese Vorraussetzung wird jedoch von der Dimethylphenol-Fraktion nicht erfüllt, bei dem üblicherweise der Anteil an p-Isopropylphenol überwiegt.

Es bestand daher die Aufgabe, ein geeignetes Verfahren zur Gewinnung von 3,4-Dimethylphenol aus der bei der destillativen Rohphenolaufarbeitung anfallenden Dimethylphenol-Fraktion zu entwickeln, dessen 3,4-Dimethylphenol/p-Isopropylphenol-Verhältnis kleiner als 1,5 ist. Die Aufgabe wird dadurch gelöst, daß die gegebenenfalls aufkonzentrierte Dimethylphenol-Fraktion mit einem 3,4-Dimethylphenol/p-Isopropylphenol-Verhältnis von mindestens 0,5 nach einer Redestillation zur Abtrennung der niedriger und der höher siedenden Bestandteile bis auf einen Restgehalt von maximal 10 Gew.-% mit 30 bis 65 Gew.-% Phenol, bezogen auf das Gemisch, vermischt, das Gemisch einer ersten ein- oder zweistufigen Kristallisation unterworfen, und der Restkristall nach destillativer Abtrennung des Phenols durch eine zweite ein- oder mehrstufige Kristallisation zur Gewinnung des 3,4-Dimethylphenol gereinigt wird.

Durch die Destillation des Kristallguts und der Mutterlauge der ersten Kristallisation kann das Phenol zurückgewonnen und in dem folgenden Zyklus wiederverwendet werden. Zur Steigerung der Ausbeute und Verbesserung der Wirtschaftlichkeit des Verfahrens werden die Mutterlaugen der einzelnen Kristallisationsstufen dem Einsatz der jeweils niedrigeren Kristallisationsstufe des folgenden Zyklus und die Tropföle der partiellen Aufschmelzung der einzelnen Stufen dem Einsatz der entsprochenden Stufe des folgenden Zyklus zugemischt.

Die höchste Aufkonzentration von 3,4-Dimethylphenol in der ersten Kristallisation wird bei einem Phenolanteil im Einsatz von etwa 50 Gew.-% erreicht.

Die Erfindung wird anhand des folgenden Beispiels näher erläutert:

Beispiel:

Bei der Destillation von Rohphenol fällt als Rückstand eine mit Dimethylphenolen angereicherte Fraktion, die durch Redestillation von leicht- und schwersiedenden Komponenten befreit wird. Die so erhaltene 3,4-Dimethylphenol-Fraktion hat folgende Zusammensetzung:

Leichtsieder 0,1 %
Phenol 0,3 %
o-Kresol 0,1 %
m,p-Kresol 0,2 %
2.4.- und 2.5-Dimethylphenol (DMP) 0,1 %
3.5-DMP und m,p-Äthylphenol 4,2 %
3.4-Dimethylphenol 36,8 %
p-Isopropylphenol 53,3 %
Trimethylphenole 2,0 %
Methyl-/Ethyl-Phenole 2,4 %
Höher siedende Bestandteile 3,0 %
nicht identifizierte Bestandteile 0,2 %.

Die Trennung kann auch mit einem Rücklaufverhältnis von mehr als 25 unter Verwendung einer mehr als 50-bödigen Kolonne nicht mehr verbessert werden.
102 kg dieser Fraktion werden mit 72,6 kg Tropföl mit 50 Gew.-% Phenol aus der ersten Kristallisationsstufe und 27,7 kg phenolfreier Mutterlauge der zweiten Kristallisationsstufe des vorangegangenen Zyklus sowie 127,7 kg des durch Destillation im vorangegangenen Zyklus wiedergewonnenen und 2 kg frischen Phenols bei Raumtemperatur gemischt. Der Phenolanteil beträgt also 50 Gew.-%.
Das Gemisch wird in einem Schrägflächenkristallisator, wie in der DE-OS 33 34 842 beschrieben, kurzzeitig bis auf -5°C gekühlt, um die Kristallisation zu initiieren. Danach wird die Kristallisation bei 0 °C weitergeführt. 165,4 kg Mutterlauge werden abgelassen und das verbleibende Kristallisat langsam auf 30°C erwärmt. Dabei fallen 72,6 kg Tropföl mit etwas 50 Gew.-% Phenol an, das für den Einsatz des nächsten Zyklus aufgehoben wird. Der Restkristall wird bei 40 °C abgeschmolzen und wie auch die Mutterlauge durch Destillation bei 50 mbar und einem Rücklaufverhältnis von 4 zur Abtrennung des Phenols destilliert. Die zurückgewonnenen 127,7 kg Phenol werden für den nächsten Zyklus aufgehoben. Der Rückstand aus der Destillation des Restkristalls der ersten Kristallisationsstufe wird in einem Schrägflächenkristallisator bis auf 5 °C gekühlt. Aus dieser zweiten Kristallisationsstufe werden 27,7 kg Mutterlauge abgezogen und für den Einsatz der ersten Kristallisationsstufe des folgenden Zyklus aufgehoben. Für die partielle Aufschmelzung wird das Kristallisat bis auf 50 °C erwärmt. Dabei werden 21,3 kg Tropföl erhalten, die dem Einsatz der zweiten Kristallisationsstufe des folgenden Zyklus zugegeben werden. Der Restkristall wird bei 65 °C aufgeschmolzen und er dritten Kristallisationsstufe zugeführt. Die Kristallisation erfolgt hier bei 35 °C. Es werden 6,2 kg Mutterlauge abgelassen und für den Einsatz der zweiten Kristallisationsstufe des folgenden Zyklus aufgehoben. Das Kristallgut wird auf 63 °C erwärmt. Die dabei gewonnenen 20 kg Tropföl werden für den Einsatz der dritten Kristallisationsstufe des folgenden Zyklus aufbewahrt.

Die Mengen und deren Konzentrationen an 3,4-Dimethylphenol, p-Isopropylphenol und Phenol bei den einzelnen Kristallisationsstufen sind in der Tabelle wiedergegeben:

### Tabelle

| | Menge Gew.-% | Konzentration Gew.-% an | | |
| | | 3,4-Dimethylphenol | p-Isopropylphenol | Phenol |
|---|---|---|---|---|
| **1. Stufe:** | | | | |
| Einsatz | 100 | 19,4 | 26,5 | 50 |
| Mutterlauge | 50 | 11,7 | 33,1 | 50 |
| Tropföl | 22 | 19,3 | 26,1 | 50 |
| Restkristall | 28 | 33,0 | 15,5 | 50 |
| **2. Stufe:** | | | | |
| Einsatz | 100 | 64,0 | 31,0 | – |
| Mutterlauge | 38 | 41,0 | 50,6 | – |
| Tropföl | 29 | 64,0 | 31,2 | – |
| Restkristall | 33 | 90,0 | 8,2 | – |
| **3. Stufe:** | | | | |
| Einsatz | 100 | 90,0 | 8,6 | – |
| Mutterlauge | 14 | 65,0 | 30,0 | – |
| Tropföl | 45 | 90,1 | 8,7 | – |
| Restkristall | 41 | 98,5 | 1,3 | – |

Die Ausbeute an technisch reinem 3,4-Dimethylphenol beträgt etwa 48 % bei dem erfindungsgemäßen Verfahren. Durch weitere Kristallisationsstufen kann die Reinheit noch erhöht werden.

Der Rückstand der ersten Kristallisationsstufe wird ausgeschleust. Er enthält 66,0 % p-Isopropylphenol und 23,2 % 3,4-Dimethylphenol. Das Konzentrationsverhältnis beträgt also nun 0,35.

Dieser Rückstand kann destillativ soweit aufkonzentriert werden, daß er dem Prozeß wieder zugeführt werden kann. Die Ausbeuten sind jedoch gering.

Es wurde nun gefunden, daß sich der Gehalt an 3,4-Dimethylphenol auch durch eine Kristallisationsstufe erhöhen läßt, wenn die Kristallisation durch Animpfen mit p-Isopropylphenol-Kristallen eingeleitet wird. Auf diese Weise kann in einer Stufe etwa 80 %iges p-Isopropylphenol als Restkristall und eine 3,4-Dimethylphenol-Fraktion mit ausreichendem Konzentrationsverhältnis als Mutterlauge gewonnen werden. Da sich in der Mutterlauge auch die Begleitstoffe anreichern, müssen diese durch eine Destillation abgetrennt werden, wie dies für den Rückstand aus der Rohphenoldestillation beschrieben worden ist.

Versuche, das Phenol der ersten Kristallisationsstufe durch andere Hilfsstoffe zu ersetzen, schlugen fehl. So führte die Verwendung von Kresolen zu hochviskosen Schmelzen, bei denen eine Stofftrennung nicht möglich ist. Die Verwendung isomerer Dimethylphenole ergab zwar eine gute Kristallbildung, aber Mutterlauge und Kristallgut hatten etwa die gleiche Zusammensetzung. Die Kristallisation ohne Hilfsstoffe führt zu einem sehr feinen, unporösen Kristallgut, das sich in dem Konzentrationsverhältnis kaum vom Einsatz unterscheidet.

Das Konzentrationsverhältnis von 0,5 ist für das erfindungsgemäße Verfahren als untere Grenze anzusehen, bei dem sich trotz Phenolzusatz bereits ein sehr weiches Kristallgut bildet. Das Konzentrationsverhältnis sollte daher vorzugsweise mindestens 0,7 betragen, damit die erste Kristallisation einstufig durchgeführt werden kann. Bei Konzentrationsverhältnissen im Bereich von 0,5 bis 0,7 ist im allgemeinen eine zweistufige erste Kristallisation mit Phenolzusatz erforderlich.

**Patentansprüche**

1. Verfahren zur Gewinnung von 3,4-Dimethylphenol aus p-Isopropylphenol enthaltenden Dimethylphenol-Fraktionen, dadurch gekennzeichnet, daß die gegebenenfalls aufkonzentrierte Dimethylphenol-Fraktion mit einem 3,4-Dimethylphenol/p-Isopropylphenol-Verhältnis von mindestens 0,5 nach einer Redestillation zur Abtrennung der niedriger und höher siedenden Bestandteile bis auf einen Restgehalt vom maximal 10 Gew.-% mit 30 bis 65 Gew.-% Phenol, bezogen auf das Gemisch, vermischt, das Gemisch einer ersten ein- oder zweistufigen Kristallisation unterworfen, und der Restkristall nach destillativer Abtrennung des Phenols durch eine zweite ein- oder mehrstufige Kristallisation zur Gewinnung des 3,4-Dimethylphenols gereinigt wird.

2. Verfahren nach Anspruch 1, dadurch gekennzeichnet, daß bei einem Konzentrationsverhältnis von mindestens 0,7 die erste Kristallisation mit Phenol einstufig ausgeführt wird.

3. Verfahren nach Anspruch 1, dadurch gekennzeichnet, daß bei einem Konzentrationsverhältnis im Bereich von 0,5 bis 0,7 die erste Kristallisation zweistufig ausgeführt wird.

4. Verfahren nach den Ansprüchen 1 bis 3, dadurch gekennzeichnet, daß das bei der destillativen Aufarbeitung des Kristalls und der Mutterlauge der ersten Kristallisation gewonnene Phenol in die erste Kristallisation des folgenden Zyklus zurückgeführt wird.

5. Verfahren nach den Ansprüchen 1 bis 4, dadurch gekennzeichnet, daß das Tropföl der jeweiligen Kristallisationsstufen eines Zyklus in die entsprechende Kristallisationsstufe des folgenden Zyklus zurückgeführt wird.

6. Verfahren nach den Ansprüchen 1 bis 5, dadurch gekennzeichnet, daß die Mutterlauge der einzelnen Kristallisationstufen der zweiten Kristallisation eines Zyklus in die vorausgehende Kristallisationsstufe des folgenden Zyklus zurückgeführt wird.

7. Verfahren nach den Ansprüchen 1 bis 6, dadurch gekennzeichnet, daß Dimethylphenol-Fraktionen mit niedrigeren Konzentrationsverhältnis durch Kristallisation, die durch Animpfen mit p-Isopropylphenol-Kristallen eingeleitet wird, das p-Isopropylphenol teilweise entzogen wird und die abgezogene Mutterlauge als Einsatzprodukt für die Redestillation verwendet wird.

**Claims**

1. A process for the recovery of 3,4-dimethylphenol from dimethylphenol fractions containing p-isopropylphenol, characterized in that the dimethylphenol fraction – concentrated where appropriate and having a ratio of 3,4-dimethylphenol to p-isopropylphenol of at least 0.5 after a redistillation to separate the lower and higher boiling constituents to a residual content of at most 10% by weight – is mixed with from 30 to 65% by weight of phenol, relative to the mixture, the mixture is subjected to a first one-stage or two-stage crystallization, and after separation of the phenol by distillation the residual crystal is purified by a second one-stage or multiple-stage crystallization to recover the 3,4-dimethylphenol.

2. A process according to Claim 1, characterized in that with a concentration ratio of at least 0.7 the first crystallization is performed with phenol in one stage.

3. A process according to Claim 1, characterized in that with a concentration ratio in the range of from 0.5 to 0.7 the first crystallization is performed in two stages.

4. A process according to Claims 1 to 3, characterized in that the phenol recovered in the processing – by distillation – of the crystal and the mother liquor of the first crystallization is fed back to the first crystallization of the following cycle.

5. A process according to Claims 1 to 4, characterized in that the drip oil of the respective crystallization stages of one cycle is fed back into the corresponding crystallization stage of the following cycle.

6. A process according to Claims 1 to 5, characterized in that the mother liquor of the individual crystallization stages of the second crystallization of one cycle is fed back into the preceding crystallization stage of the following cycle.

7. A process according to Claims 1 to 6, characterized in that dimethylphenol fractions having lower concentration ratio(s) (are formed?) through crystallization, which is seeded by inoculation with p-isopropylphenol crystals, the p-isopropylphenol is partially removed and the mother liquor drawn off is used as a charging product for redistillation.

**Revendications**

1. Procédé pour l'obtention de 3,4-diméthylphénol à partir de fractions de diméthylphénol contenant du p-isopropylphénol, caractérisé en ce que la fraction de diméthylphénol, éventuellement concentrée, avec un rapport 3,4diméthylphénol/p-isopropylphénol d'au moins 0,5 est mélangée, après une redistillation en vue de la séparation des constituants bouillant plus bas et plus haut jusqu'à une teneur résiduelle de 10% en poids au plus, avec 30 à 65% en poids de phénol par rapport au mélange, que le mélange est soumis à une première cristallisation en une ou deux étapes et qu'après une séparation distillatoire du phénol, les cristaux résiduels sont purifiés par une deuxième cristallisation en une ou plusieurs étapes en vue de l'obtention du 3,4-diméthylphénol.

2. Procédé selon la revendication 1, caractérisé en ce qu'avec un rapport de concentration d'au moins 0,7, la première cristallisation est conduite en une étape avec du phénol.

3. Procédé selon la revendication 1, caractérisé en ce qu'avec un rapport de concentration dans la gamme de 0,5 à 0,7, la première cristallisation est conduite en deux étapes.

4. Procédé selon les revendications 1 à 3, caractérisé en ce que le phénol obtenu lors du traitement distillatoire des cristaux et des eaux-mères de la première cristallisation est renvoyé vers la première cristallisation du cycle suivant.

5. Procédé selon les revendications 1 à 4, caractérisé en ce que l'huile d'égouttage des étapes de cristallisation respectives d'un cycle est renvoyée dans l'étape de cristallisation correspondante du cycle suivant.

6. Procédé selon les revendications 1 à 5, caractérisé en ce que les eaux-mères des étapes de cristallisation individuelles de la deuxième cristallisation d'un cycle sont renvoyées dans l'étape de cristallisation qui précède du cycle suivant.

7. Procédé selon les revendications 1 à 6, caractérisé en ce que le p-isopropylphénol est partiellement extrait de fractions de diméthylphénol avec un rapport de concentration inférieur, par une cristallisation qui est amorcée par ensemencement avec des cristaux de p-isopropylphénol et que les eaux-mères soutirées sont utilisées comme charge pour la redistillation.